(19) Europäisches Patentamt
European Patent Office
Office européen des brevets

(11) EP 4 166 560 A1

(12) EUROPEAN PATENT APPLICATION

(43) Date of publication:
19.04.2023 Bulletin 2023/16

(21) Application number: 21382920.3

(22) Date of filing: 13.10.2021

(51) International Patent Classification (IPC):
*C07F 7/08* (2006.01) *A61K 31/695* (2006.01)
*A61P 31/12* (2006.01)

(52) Cooperative Patent Classification (CPC):
C07F 7/0814; A61P 31/12

(84) Designated Contracting States:
AL AT BE BG CH CY CZ DE DK EE ES FI FR GB GR HR HU IE IS IT LI LT LU LV MC MK MT NL NO PL PT RO RS SE SI SK SM TR
Designated Extension States:
BA ME
Designated Validation States:
KH MA MD TN

(71) Applicants:
- Consejo Superior de Investigaciones Cientificas
28006 Madrid (ES)
- Universitat de Valéncia
46010 Valencia (ES)

(72) Inventors:
- PADRON PEÑA, Juan Ignacio
38206 San Cristobal de La Laguna (ES)
- SINKA, María Victoria
38206 San Cristobal de La Laguna (ES)
- SANJUÁN VERDEGUER, Rafael
46010 Valencia (VALENCIA) (ES)
- DUFLOO, Jérémy
75724 Paris (FR)
- Schwartz, Olivier
75724 Paris (FR)

(74) Representative: Pons
Glorieta Rubén Darío 4
28010 Madrid (ES)

(54) TRI-SUBSTITUTED TETRAHYDROFURANS AND USE THEREOF

(57) The invention relates to new tri-substituted tetrahydrofurans of formula (I) and their use as antiviral agent in the treatment and/or prevention of coronavirus infections and more particularly SARS-CoV-2 infection.

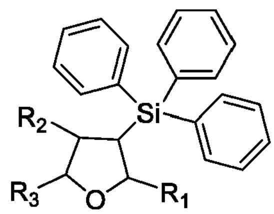

(I)

EP 4 166 560 A1

## Description

The invention relates to new tri-substituted tetrahydrofurans and their use as antiviral agents in the treatment and/or prevention of coronavirus infections, including COVID-19.

## BACKGROUND ART

Type-2 coronavirus causing severe acute respiratory syndrome (SARS-CoV-2) is the causative agent of COVID-19. The emergence of this virus at the end of 2019 has posed a huge biomedical challenge. Other related coronaviruses have previously emerged in the human population, such as SARS coronavirus and MERS coronavirus. Finding effective antivirals against SARS-CoV-2 and other coronaviruses is therefore a global priority.

To date, few drugs with demonstrable therapeutic effect in COVID-19 patients have been described, and their effects have been modest at best. A compound with a slight ability to improve the prognosis of these patients is Remdesivir, a nucleoside analogue exhibiting broad-range antiviral activity, for which an $IC_{50}$ against SARS-CoV-2 on the order of 1 μM was shown (Pizzorno, A. et al. In vitro evaluation of antiviral activity of single and combined repurposable drugs against SARS-CoV-2. Antiviral Res 181, 104878 (2020)). In addition, there are numerous compounds with potential anti-SARS-CoV-2 effect *in vitro,* some of which are in the pre-clinical or clinical research phase. This includes a potent *in vitro* activity has been described for Aplidin (plitidepsin), a compound belonging to the didemnin family that was initially developed as an antitumoral (White, K. M. et al. Plitidepsin has potent preclinical efficacy against SARS-CoV-2 by targeting the host protein eEF1A. Science 371, 926-931 (2021).

Therefore, due to the need for safe and effective antiviral agents with a wide-spectrum of anti-viral activity, especially against coronaviruses, the present invention provides compounds that could be useful as antiviral agents and, more particularly, against coronavirus infections.

## SUMMARY OF THE INVENTION

The present invention is based on the discovery of tri-substituted five-membered ring oxacycles, in particular, tetrahydrofurans, which are active compounds against coronaviruses and other types of viruses.

The first aspect of the present invention relates to a compound of formula (I):

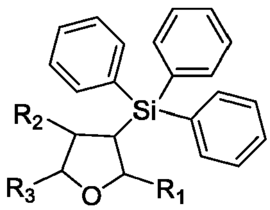

(I)

a isomer or a solvate thereof, wherein

$R_1$ is selected from $CH_3$ and $(CH_2)_nX$, being n and integer between 1 and 4 and X an halogen,
$R_2$ is selected from H and phenyl,
$R_3$ is selected from H, phenyl, benzyl and $C_1$-$C_4$ alkyl,
wherein one of $R_2$ and $R_3$ is H and the other one is different from H and
with the proviso that when $R_3$ is $C_1$-$C_4$ alkyl, $R_1$ is $(CH_2)_nX$.

The above structure, as defined, comprises a phenyl group in addition to the phenyl groups of the $SiPh_3$ substituent and/or an alkyl group substituted by an halogen ($(CH_2)_nX$, group).

The term "halogen" refers to fluoride, chloride, bromide or iodine.

The term "benzyl" refers to a $-CH_2Ph$ group, wherein Ph represents a phenyl group.

The term "$C_1$-$C_4$ alkyl" in the present invention refers to linear or branched saturated hydrocarbon chain that have 1 to 4 carbon atoms, for example, methyl, ethyl, n-propyl, i-propyl, n-butyl, sec-butyl, tert-butyl.

In a preferred embodiment, $R_1$ is $CH_3$.

In a preferred embodiment, $R_2$ is H and $R_3$ is selected from phenyl and benzyl, more preferably $R_3$ is benzyl.

In a preferred embodiment, $R_3$ is H and $R_2$ is phenyl.

In a preferred embodiment, $R_3$ is $C_1$-$C_4$ alkyl, more preferably, iso-butyl and $R_1$ is $(CH_2)_nX$, being n equals 3

and/or X is Cl.

Unless otherwise stated, the compound of formula (I) is intended to include compounds that differ only in the presence of one or more isotopically enriched atoms. For example, compounds having the present structures except for the substitution of a hydrogen atom for a deuterium atom or a tritium atom, or the substitution of a carbon atom for a carbon atom enriched in $^{13}C$ or $^{14}C$ or a nitrogen atom enriched in $^{15}N$ fall within the scope of this invention.

It must be understood that compound of formula (I) encompasses all the isomers of said compound, i.e. all the geometric, tautomeric and optical forms, and their mixtures (for example, racemic mixtures). The present invention includes, within its scope, all the possible diastereomers, including their mixtures. The different isomeric forms may be separated or resolved there between using conventional methods or any given isomer can be obtained using conventional synthetic methods or by means of stereospecific, stereoselective or asymmetric synthesis.

The term "racemic mixture," "racemic compound" or "racemate" refers to an equimolecular mixture of the two enantiomers of one compound.

The term "enantiomer" is used to describe one of a pair of molecular isomers which are mirror images of each other and non-superimposable.

The term "diastereomers" or "diastereoisomers" as used herein refers to stereoisomers that are not enantiomers. The diastereomers have different configurations at one or more of the equivalent (related) stereocenters and are not mirror images of each other.

In certain embodiments, the compound of formula (I) is a mixture of stereoisomers, e.g., a mixture of diastereomers and/or enantiomers, e.g., a racemic mixture. In another such embodiment, the compound is a mixture of diastereomers. In another such embodiment, the compound is a mixture of enantiomers.

In a preferred embodiment, compound of formula (I) is a diastereomer selected from

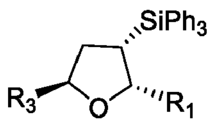 ,

(I')

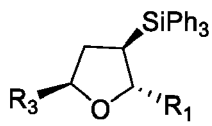

(I")

or combinations thereof ($R_2$ is H in I' and I"). Preferably, $R_1$ is $CH_3$ in formulas (I') and (I"); and when $R_1$ is $CH_3$, $R_3$ is phenyl or benzyl, preferably benzyl.

In these diastereomers of formula (I') and (I"), the substituents $R_1$ and $R_3$ are in relative position *trans*. The difference between these two diastereomers is in that they have different configurations at the carbon atom bonded to the Si atom.

In an embodiment, the compound of formula (I) is a racemic mixture of the diastereomer of formula (I').

In another embodiment, the compound of formula (I) is a racemic mixture of the diastereomer of formula (I").

In a preferred embodiment, the compound of formula (I) is a mixture of the diastereomers of formula (I') and (I") as represented above. More preferably, the compound of formula (I) is an equimolecular mixture of said diastereomers of formula (I') and (I").

In a preferred embodiment, compound of formula (I) is a mixture of said diastereomers, more preferably an equimolecular mixture, of formula (I') and (I") being each diasteromer a racemic mixture. Even more preferably, compound of formula (I) is an equimolecular mixture of diastereomers of formula (I') and (I"), wherein $R_1$ is $CH_3$ and $R_3$ is benzyl, being each diastereomer a racemic mixture.

In a preferred embodiment, the compound of formula (I) is the isomer of formula (I") ($R_2$ is H):

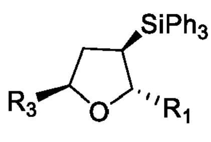 (I")

wherein $R_3$ is $C_1$-$C_4$ alkyl.

In a preferred embodiment, the compound of formula (I) is the isomer of formula (I‴) ($R_3$ is H in I‴):

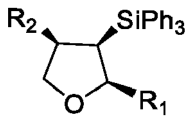

(I''')

Preferably, $R_1$ is $CH_3$ in the formula (I''').

In a preferred embodiment, the isomer of formula (I''') is a racemic mixture.

In a preferred embodiment, the compound of formula (I) is selected from the next ones:

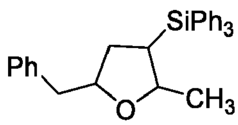
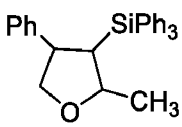
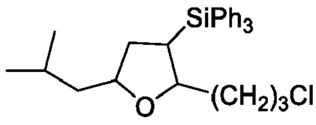

wherein Ph represents a phenyl group.

More preferably, the compound of formula (I) is selected from the next isomers:

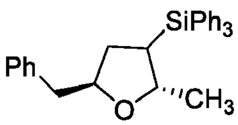
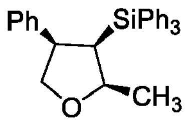
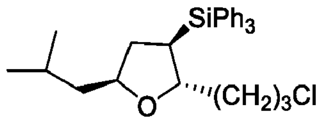

wherein Ph represents a phenyl group. Preferably, these compounds are in form of a racemic mixture.

The next formula:

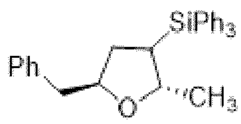

represents any of the two possible diastereomers depending on the stereochemistry of the carbon atom bonded to the $SiPh_3$ group, or a mixture thereof.

The compound of formula (I) may be in crystalline form, either as free compound or as solvate (e.g.: hydrate), and it is understood that both forms fall within the scope of the present invention. Solvation methods are generally known in the state of the art. Suitable solvates are pharmaceutically acceptable solvates. In a particular embodiment, the solvate is a hydrate.

A second aspect of the invention refers to a compound of formula (I), as defined in the first aspect of the invention, for use as a medicament.

A third aspect of the invention refers to a compound of formula (I), as defined in the first aspect of the invention, for use as antiviral agent, more preferably, in the treatment and/or prevention of coronavirus infection.

Coronavirus infection preferably refers to SARS-CoV-2, human coronavirus OC43, transmissible gastroenteritis virus (TGEV), or murine hepatitis virus (MHV) infection. More preferably, it is a SARS-CoV-2 infection.

The term "treatment or prevention" as used herein, unless otherwise indicated, relates to reversing, alleviating and inhibiting the progress of, or preventing the disorder or condition to which it applies in such terms, one or more symptoms of such disorder or condition.

In another aspect, the invention relates to a method for the prevention and/or treatment of coronavirus infection in a subject, preferably SARS-CoV-2 infection, comprising the administration to said subject of a therapeutically effective amount of a compound of formula (I) as defined in the first aspect of the present invention.

In another aspect, the invention relates to the use of a compound of formula (I) as defined in the first aspect of the present invention for the preparation of a medicament for prevention and/or treatment of coronavirus infection.

Another aspect of the invention refers to a pharmaceutical composition comprising the compound of formula (I), as described above, isomers or a solvate thereof.

The pharmaceutical composition preferably includes at least one excipient, adjuvant and/or a pharmaceutically acceptable carrier.

The term "carrier" relates to a dilute, adjuvant or excipient with which the main ingredient is administered. Such pharmaceutical carriers may be sterile liquids, such as water and oils, including those derived from oil, animal, vegetable or synthetic, such as peanut oil, soybean oil, mineral oil, sesame seed oil and similar. Water or aqueous saline solutions and aqueous dextrose and glycerol solutions are preferably used as carriers, particularly for injectable solutions. Preferably, the carriers of the invention are approved by the regulatory agency of a state government or a federal government, or are enumerated in the United States Pharmacopoeia, in the European Pharmacopoeia or other pharmacopoeia recognised in general for use in animals and, more particularly, in humans.

A last aspect of the present invention refers to the pharmaceutical composition comprising the compound of formula (I), as described above, isomers or a solvate thereof, for use in the prevention and/or treatment of a virus infection, preferably coronavirus infection and, even more preferably, SARS-CoV-2, human coronavirus OC43, TGEV, or MHV infection.

The pharmaceutical composition comprises a therapeutically effective amount of the compound of formula (I), isomers or solvates thereof that must be administered (also referred to herein as therapeutically amount effective thereof).

The therapeutically amount effective of the compound of formula (I), in addition to their dosage for treating a pathological condition with said compound, will depend on a number of factors, including age, the condition of the patient, the severity of the disease, administration route and frequency, the modular compound to be used, etc.

The pharmaceutical compositions of this invention can be used on their own or jointly with other drugs to provide combined therapy. The other drugs can form part of the same pharmaceutical composition or be provided as a separate pharmaceutical composition, to be administered simultaneously or at different intervals. Examples of pharmaceutical compositions include any solid composition (tablets, pills, capsules, granules, etc.) or liquid composition (solutions, suspensions or emulsions) for oral, topical or parenteral administration.

Unless otherwise defined, all technical and scientific terms used herein have the same meaning as commonly understood by one of ordinary skilled in the art to which this invention belongs. Methods and materials similar or equivalent to those described herein can be used in the practice of the present invention. Throughout the description and claims the word "comprise" and its variations are not intended to exclude other technical features, additives, components, or steps. Additional objects, advantages and features of the invention will become apparent to those skilled in the art upon examination of the description or may be learned by practice of the invention. The following examples, are provided by way of illustration and are not intended to be limiting of the present invention.

## BRIEF DESCRIPTION OF THE DRAWINGS

**Fig. 1. Automated real-time microscopy quantitation of ST cell viability at different concentrations of SPL-0 in the presence (a) or absence (b) of TGEV infection.** Dots indicate experimental data (average of two technical replicates). The line indicates the inferred dose-response function.

**Fig. 2. Automated real-time microscopy quantitation of ST cell viability at different concentrations of SPL-4 in the presence (a) or absence (b) of TGEV infection.** Dots indicate experimental data (average of two technical replicates). The line indicates the inferred dose-response function.

**Fig. 3. Automated real-time microscopy quantitation of ST cell viability at different concentrations of SPL-6 in the presence (a) or absence (b) of TGEV infection.** Dots indicate experimental data (average of two technical replicates). The line indicates the inferred dose-response function.

**Fig. 4. Automated real-time microscopy quantitation of ST cell viability at different concentrations of SPL-1 in the presence (a) or absence (b) of TGEV infection.** Dots indicate experimental data (average of two technical replicates). The line indicates the inferred dose-response function.

**Fig. 5. Automated real-time microscopy quantitation of ST cell viability at different concentrations of SPL-5 in the presence (a) or absence (b) of TGEV infection.** Dots indicate experimental data (average of two technical replicates). The line indicates the inferred dose-response function.

**Fig. 6. TGEV titration in ST cell cultures treated with different concentrations of SPL-0.** Dots indicate experimentally determined viral titers relative to an untreated control (average of three technical replicates). The line indicates the inferred dose-response function.

**Fig. 7. Quantitation of TGEV RNA by RT-qPCR in ST cell cultures treated with different concentrations of**

**SPL-0.** Dots indicate experimentally determined RNA concentrations relative to an untreated control (average of three technical replicates). The line indicates the inferred dose-response function.

**Fig. 8. Effect of SPL-0 on SARS-CoV-2 infection in VeroE6 cells.** Viral infection was quantified by immunofluorescence in cultures treated with different concentrations of SPL-0 (a). The percentage of viable cells in uninfected cultures was determined by the MTT assay (b). Dots indicate the experimentally determined percentage of infected cells. The line indicates the inferred dose-response function.

**Fig. 9. Effect of SPL-0 on SARS-CoV-2 infection in A549 cells.** Viral infection was quantified by immunofluorescence in cultures treated with different concentrations of SPL-0 (a). The percentage of viable cells in uninfected cultures was determined by the MTT assay (b). Dots indicate the experimentally determined percentage of infected cells. The line indicates the inferred dose-response function.

**Fig. 10. CVB3 titration in HeLa cell cultures treated with different concentrations of SPL-0.** Dots indicate experimentally determined viral titers relative to an untreated control (average of three technical replicates). The line indicates the inferred dose-response function.

## Examples

Compounds **SPL-0, SPL-4** and **SPL-6** within the present invention have been synthetized. Also, compounds **SPL-1** and **SPL-5,** which are not within the present invention, have been synthetized for comparative purposes. The chemical structure of all these compounds is represented in the examples below.

### Example 1: Synthesis of compound SPL-0

This compound (SPL-0) was obtained in two reaction steps, starting from the corresponding commercially available epoxide, and with an overall yield of 51%.

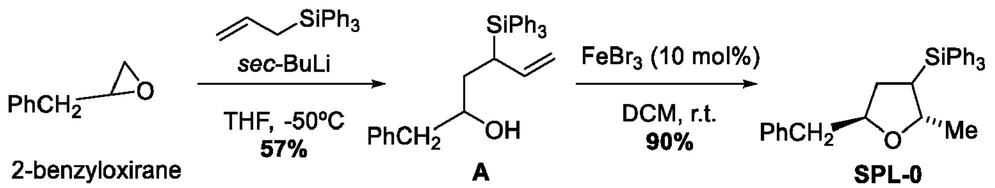


2-benzyloxirane **A** **SPL-0**

**Scheme 1.** Synthesis of tri-substituted five-membered ring oxacycle **SPL-0** ((2,5-*trans*-5-benzyl-2-methyltetrahydrofuran-3-yl)triphenylsilane).

### 1-phenyl-4-(triphenylsilyl)hex-5-en-2-ol (A)

To a well-stirred solution of allyltriphenylsilane (2.59 g, 8.46 mmol, 1.0 equiv.) in 42 mL of dry tetrahydrofuran (THF) (0.2 M *vs* allyltriphenylsilane), cooled to -78 ºC and under $N_2$ atmosphere, was added sec-butyllithium 1.4 M (6.0 mL, 8.46 mmol, 1.0 equiv.). Then the system was allowed to reach -50 ºC and this temperature was kept along 2 hours. After that, the system was cooled again to -78 ºC and 2-benzyloxirane (1.14 g, 8.46 mmol, 1.0 equiv.) was added dropwise, solved in a little amount of dry THF. The reaction was stirred at -78ºC until analysis via Thin Layer Chromatography (TLC) showed complete formation of alcohol **A.** It was quenched by addition of a mixture of *n*-hexane:ethyl ether:saturated aqueous $NH_4Cl$ (2:2:1), being the volume of this mixture = 25 times the millimole of allyltriphenylsilane and extracted with ethyl ether. The combined organic layers were dried over anhydrous $MgSO_4$, filtered, and the solvent was removed under reduced pressure. This crude reaction mixture was purified by flash silica gel column chromatography (n-hexane/EtOAc 90:10 solvent system) to afford 2.10 g of alcohol **A** (4.82 mmol, 57% yield). Spectral data was consistent with the known product (Cruz, D. A.; Sinka, V.; Martin, V. S.; Padrón, J. I. J. Org. Chem. 2018, 83, 12632-12647).

**$^1$H NMR ($CDCl_3$, 600 MHz)** (mixture *syn*/*anti* (1:0.7)) δ 7.60-7.53 (m, 12H), 7.41 (m, 6H), 7.36 (m, 12H), 7.28 (m, 4H), 7.21 (m, 2H), 7.15 (m, 4H), 5.89 (m, 1H), 5.71 (m, 1H), 5.06-4.88 (m, 4H), 3.91 (m, 2H), 2.99 (brt, *J* = 10.3 Hz, 1H), 2.89 (dd, *J* = 4.0 & 13.7 Hz, 1H), 2.72-2.64 (m, 2H), 2.64-2.58 (dd, *J* = 8.9 & 13.7 *Hz,* 1H), 2.54 (dd, *J* = 8.5 & 13.6 Hz, 1H), 1.95 (ddd, *J* = 2.6, 6.8 & 14.0 Hz, 1H), 1.91-1.82 (m, 2H), 1.74 (m, 1H), 1.69 (m, 1H), 1.53 (m, 1H).

**$^{13}$C NMR ($CDCl_3$, 150 MHz)** (mixture *syn*/*anti* (1:0.7)) δ 139.3 (CH), 138.8 (C), 138.6 (C), 138.3 (CH), 136.2 (6xCH), 136.1 (6xCH), 133.8 (3xC), 133.4 (3xC), 129.5 (3xCH), 129.4 (3xCH), 129.3 (2xCH), 129.3 (2xCH), 128.5

(2xCH), 128.5 (2xCH), 127.8 (6xCH), 127.8 (6xCH), 126.4 (CH), 126.3 (CH), 115.3 ($CH_2$), 115.1 ($CH_2$), 73.6 (CH), 70.4 (CH), 44.5 ($CH_2$), 42.6 ($CH_2$), 36.3 ($CH_2$), 36.2 ($CH_2$), 30.5 (CH), 237.8 (CH).

**HRMS (ESI$^+$):** *m/z* $[M+Na]^+$ calcd. for $C_{30}H_{30}OSi$: 457.1964; found: 457.1959.

**2,5-*trans*-5-benzyl-2-methyltetrahydrofuran-3-yl)triphenylsilane (SPL-0)**

To a well-stirred solution of *bis*-homoallylsilyl alcohol **A** (300 mg, 0.69 mmol, 1.0 equiv.) in 6.9 mL of dry dichloromethane (DCM) (0.1 M) at room temperature under $N_2$ atmosphere, was added $FeBr_3$ (20.4 mg, 0.069 mmol, 0.10 equiv.). Once the reaction was complete, the process was quenched by addition of water. The aqueous phase was extracted with dichloromethane. The combined organic layers were dried over anhydrous $MgSO_4$, filtered, and the solvent was removed under reduced pressure. The crude reaction mixture was purified by flash silica gel column chromatography (n-hexane/EtOAc 95:5 solvent system) to give 270 mg of **SPL-0** (0.62 mmol, 90% yield).

**$^1$H NMR ($CDCl_3$, 500 MHz)** (diastereoisomeric mixture (1:1)) δ 7.53 (m, 11H), 7.42 (m, 6H), 7.38-7.33 (m, 12H), 7.29-7.22 (m, 5H), 7.21-7.14 (m, 4H), 7.11 (m, 2H), 4.29 (m, 1H), 4.22 (m, 1H), 4.06 (m, 1H), 3.87 (m, 1H), 2.92 (dd, *J* = 13.7 & 5.9 Hz, 1H), 2.86 (dd, *J* = 13.5 & 5.5 Hz, 1H), 2.71 (dd, *J* = 13.6 & 6.7 Hz, 1H), 2.59 (dd, *J* = 13.7 & 7.0 Hz, 1H), 2.28 (ddd, *J* = 12.7, 7.6 & 5.6 Hz, 1H), 2.06 (m, 2H), 1.95-1.88 (ddd, *J* = 12.1 8.9 & 7.9 Hz, 1H), 1.88-1.81 (dd, *J* = 19.2 & 9.8 Hz, 1H), 1.74 (dt, *J* = 12.2 & 9.0 Hz, 1H), 1.11 (d, *J* = 5.9 Hz, 3H), 1.07 (d, *J* = 5.9 Hz, 3H).

**$^{13}$C NMR ($CDCl_3$, 150 MHz)** (diastereoisomeric mixture (1:1)) δ 138.7 (C), 138.6 (C), 136.0 (6 x CH), 135.9 (6 × CH), 133.9 (3 x C), 133.8 (3 × C), 129.6 (4 × CH), 129.3 (4 × CH), 129.2 (4 × CH), 128.2 (2 × CH), 128.1 (2 × CH), 127.9 (5 × CH), 127.9 (5 × CH), 126.1 (CH), 126.0 (CH), 78.8 (CH), 78.7 (CH), 78.2 (CH), 77.3 (CH), 42.3 ($CH_2$), 41.5 ($CH_2$), 36.3 ($CH_2$), 35.1 ($CH_2$), 32.9 (CH), 30.7 (CH), 22.6 ($CH_3$), 22.0 ($CH_3$).

**HRMS (ESI$^+$)**: *m/z* $[M+Na]^+$ calcd. for $C_{30}H_{30}OSi$: 457.1964; found: 457.1960.

**Example 2: Synthesis of compound SPL-1**

Following the general procedure applied to SPL-0, this compound was synthesized in two steps with an overall yield of 36%.

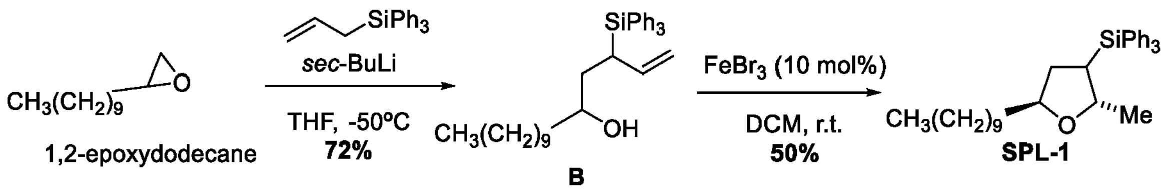


**Scheme 2.** Synthesis of tri-substituted five-membered ring oxacycle **SPL-1** ((2,5-*trans*-5-decyl-2-methyltetrahydrofuran-3-yl)triphenylsilane).

**3-(triphenylsilyl)pentadec-1-en-5-ol (B)**

To a well-stirred solution of allyltriphenylsilane (2.94 g, 9.77 mmol, 1.2 equiv.) in 49 mL of dry THF (0.2 M *vs* allyltriphenylsilane), cooled to -78 ºC and under $N_2$ atmosphere, was added sec-butyllithium 1.4 M (7.0 mL, 9.77 mmol, 1.2 equiv.). Then the system was allowed to reach -50 ºC and this temperature was kept along 2 hours. After that, the system was cooled again to -78 ºC and commercially available 1,2-epoxydodecane (1.78 mL, 8.14 mmol, 1.0 equiv.) was added dropwise, solved in a little amount of dry THF. The reaction was stirred at -78 ºC until analysis via TLC showed complete formation of alcohol **B.** It was quenched by addition of a mixture of n-hexane:ethyl ether:saturated aqueous $NH_4Cl$ (2:2:1), being the volume of this mixture = 25 times the millimole of allyltriphenylsilane and extracted with ethyl ether. The combined organic layers were dried over anhydrous $MgSO_4$, filtered and the solvent was removed under reduced pressure. This crude reaction mixture was purified by flash silica gel column chromatography (n-hexane/EtOAc 90:10 solvent system) to obtain 2.84 g of alcohol **B** (5.86 mmol, 72% yield). Spectral data was consistent with the known product.

**$^1$H NMR ($CDCl_3$, 400 MHz)** (mixture *syn*/*anti* (1:1)) δ 7.60-7.55 (dt, *J* = 1.5 & 8.0 Hz, 12H), 7.44-7.38 (m, 6H), 7.38-7.32 (m, 12H), 5.94-5.83 (m, 1H), 5.80-5.68 (m, 1H), 5.04-4.93 (m, 4H), 3.76-3.61 (m, 2H), 2.96 (m, 1H), 2.61 (m, 1H), 1.87 (ddd, *J* = 2.5 & 6.1 & 14.0 Hz, 1H), 1.79-1.69 (m, 2H), 1.67-1.63 (m, 1H), 1.63-1.58 (m, 1H), 1.41-1.18 (m, 36H), 0.90-0.85 (m, 6H).

**$^{13}C$ NMR ($CDCl_3$, 150 MHz)** (mixture *syn*/*anti* (1:1)) δ 139.6 (CH), 138.6 (CH), 136.2 (6 x CH), 136.1 (6 x CH), 133.8 (3 x C), 133.6 (3 x C), 129.5 (3 x CH), 129.4 (3 x CH), 127.8 (6 x CH), 127.7 (6 x CH), 115.2 ($CH_2$), 114.9 ($CH_2$), 73.2 (CH), 69.7 (CH), 38.0 ($CH_2$), 37.1 ($CH_2$), 36.7 ($CH_2$), 36.2 ($CH_2$), 31.9 (2×$CH_2$), 30.9 (CH), 29.6 (8 x $CH_2$), 29.3 (2 x $CH_2$), 27.9 (CH), 25.9 ($CH_2$), 25.3 ($CH_2$), 22.7 (2 x $CH_2$), 14.1 (2 x $CH_3$).

**HRMS ($ESI^+$):** *m/z* $[M+Na]^+$ calcd. for $C_{33}H_{44}OSi$: 507.3059; found: 507.3050.

**2,5-*trans*-(5-decyl-2-methyltetrahydrofuran-3-yl)triphenylsilane(SPL-1)**

To a well-stirred solution of *bis*-homoallylsilyl alcohol **B** (1.0 equiv.) in dry DCM (0.1 M) at room temperature under $N_2$ atmosphere, was added $FeBr_3$ (0.10 equiv.). Once the reaction was complete, the process was quenched by addition of water. The aqueous phase was extracted with DCM. The combined organic layers were dried over anhydrous $MgSO_4$, filtered, and the solvent was removed under reduced pressure. The crude reaction mixture was purified by flash silica gel column chromatography (n-hexane/EtOAc 95:5 solvent system) to afford **SPL-1.**

**$^1H$ NMR ($CDCl_3$, 500 MHz)** (diastereoisomeric mixture (1:1)) δ 7.56 (m, 12H), 7.45-7.34 (m, 18H), 4.26 (dq, *J* = 6.0 & 8.9 Hz, 1H), 4.06 (dq, *J* = 5.9 & 9.5 Hz, 1H), 3.93 (m, 1H), 3.53 (quint, *J* = 6.7 Hz, 1H), 2.34 (ddd, J = 5.3, 7.7 & 12.7 Hz, 1H), 2.11 (m, 1H), 1.94 (m, 3H), 1.63 (dt, J = 9.4 & 12.2 Hz, 1H), 1.50-1.36 (m, 2H), 1.31-1.21 (m, 34H), 1.96 (d, *J* = 6.0 Hz, 3H), 1.08 (d, *J* = 6.1 Hz, 3H), 0.88 (t, *J* = 7.0 *Hz*, 6H).

**$^{13}C$ NMR ($CDCl_3$, 125 MHz)** (diastereoisomeric mixture (1:1)) δ 136.0 (6 x CH), 135.9 (6 x CH),134.0 (3 x C), 133.9 (3 x C), 129.6 (6 x CH), 127.9 (12 x CH), 78.5 (CH), 78.4 (CH), 77.8 (CH), 77.2 (CH), 37.0 ($CH_2$), 36.1 ($CH_2$), 35.9 ($CH_2$), 35.5 ($CH_2$), 33.0 (CH), 31.9 (2 × $CH_2$), 30.9 (CH), 29.8 ($CH_2$), 29.7 ($CH_2$), 29.6 (6 × $CH_2$), 29.3 (2 × $CH_2$), 26.2 (2 × $CH_2$), 22.8 ($CH_3$), 22.7 (2 × $CH_2$), 22.0 ($CH_3$), 14.1 (2 × $CH_3$)

**HRMS ($ESI^+$):** *m/z* $[M+Na]^+$ calcd. for $C_{33}H_{44}OSi$: 507.3059; found: 507.3051.

**Example 3: Synthesis of compound SPL-4**

This compound (SPL-4) was synthesized in two chemical steps from the commercially available 2-phenyloxirane with an overall yield of 4% (Scheme 3).

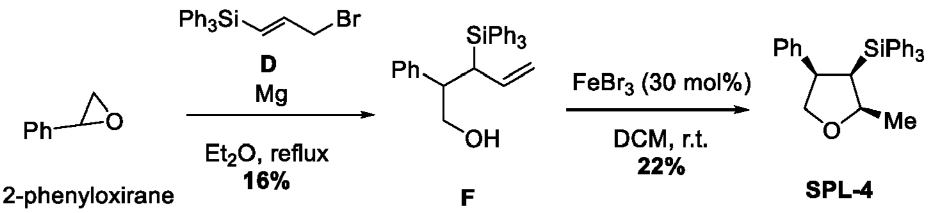

2-phenyloxirane F SPL-4

**Scheme 3.** Synthesis of tri-substituted five-membered ring oxacycle **SPL-4** ((2,3,4-*cis*-2-methyl-4-phenyltetrahydrofuran-3-yl)triphenylsilane.

**2-phenyl-3-(triphenylsilyl)pent-4-en-1-ol (F)**

Following the procedure described by Corriu (a) Corriu, R.; Masse, J. *J. Organomet. Chem.* **1973,** 57, C5-C8. b) Corriu, R. J. P.; Masse, J.; Samate, D. *J. Organomet. Chem.* **1975,** 93, 71-80), to a solution of Mg (56 mg, 2.32 mmol, 1.4 equiv.) in 5.5 mL of dry $Et_2O$ (0.3 M) was added allyl bromide **D** (758 mg, 2.00 mmol, 1.2 equiv.) dissolved in 2.0 mL of dry $Et_2O$ (1.0 M). The reaction mixture was heated to reflux for 2 h. Then, 2-phenyloxirane (200 mg, 1.66 mmol, 1.0 equiv.) was dissolved in 1.7 mL of $Et_2O$ (1.0 M) and added dropwise at the refluxing temperature. Once the reaction was complete (checked by TLC), it was allowed to cool to room temperature, filtered through a pad of Celite and quenched with saturated aqueous $NH_4Cl$. The mixture was extracted with $Et_2O$ and the organic phase was dried over anhydrous $MgSO_4$, filtered and concentrated under reduced pressure. This crude reaction mixture was purified by flash silica gel column chromatography (n-hexane/EtOAc 90:10 solvent system) to give 111 mg of *bis*-homoallylsilyl alcohol **F** (0.26 mmol, 16% yield).

**2,3,4-*cis*-2-methyl-4-phenyltetrahydrofuran-3-yl)triphenylsilane (SPL-4)**

To a well-stirred solution of *bis*-homoallylsilyl alcohol **F** (70 mg, 0.17 mmol, 1.0 equiv.) in 1.7 mL of dry DCM (0.1 M) at room temperature under $N_2$ atmosphere, was added $FeBr_3$ (15 mg, 0.051 mmol, 0.30 equiv.). Once the reaction was complete, the process was quenched by addition of water. The aqueous phase was extracted with DCM.

The combined organic layers were dried over anhydrous $MgSO_4$, filtered and the solvent was removed under reduced pressure. The crude reaction mixture was purified by flash silica gel column chromatography (n-hexane/EtOAc 95:5 solvent system) to afford 15 mg of **SPL-4** (0.036 mmol, 22 % yield).

**$^1$H NMR ($CDCl_3$, 400 MHz)** δ 7.49 (m, 6H), 7.39 (m, 3H), 7.31 (m, 6H), 7.19 (m, 3H), 7.09 (m, 2H), 4.35 (dq, *J* = 8.4 & 5.9 Hz, 1H), 3.76 (m, 2H), 3.43 (m, 1H), 2.05 (dd, *J* = 8.4 & 7.2 Hz, 1H), 1.25 (d, J = 5.9 Hz, 3H).

**$^{13}$C NMR ($CDCl_3$, 100 MHz)** δ 145.7 (C), 136.1 (6 x CH), 133.6 (3 x C), 129.6 (3 x CH), 128.4 (3 x CH), 127.8 (6 x CH), 127.6 (CH), 126.2 (CH), 79.0 (CH), 75.3 ($CH_2$), 50.0 (CH), 41.5 (CH), 22.2 ($CH_3$).

**HRMS (ESI$^+$):** m/z $[M+Na]^+$ cald. for $C_{29}H_{28}ONaSi$: 443.1807; found: 443.1805.

## Example 4: Synthesis of compound SPL-5

This compound (SPL-5) was synthesized in two chemical steps from the commercially available 2-benzyloxirane with an overall yield of 35% (Scheme 4).

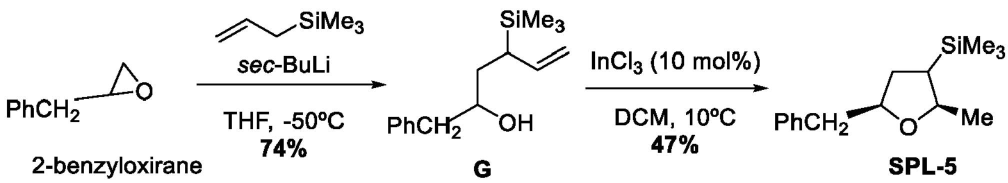

2-benzyloxirane G SPL-5

**Scheme 4.** Synthesis of tri-substituted five-membered ring oxacycle **SPL-5** ((2,5-*cis*-2-methyltetrahydrofuran-3-yl)trimethylsilane.

### 1-phenyl-4-(trimethylsilyl)hex-5-en-2-ol (G)

To a well-stirred solution of allyltrimethylsilane (0.59 mL, 3.72 mmol, 2.5 equiv.) in 19 mL of dry THF (0.2 M *vs* allyltrimethylsilane), cooled to -78 ºC and under $N_2$ atmosphere, was added sec-butyllithium 1.4 M (2.7 mL, 3.72 mmol, 2.5 equiv.). Then the system was allowed to reach -50 ºC and this temperature was kept along 2 hours. After that, the system was cooled again to -78 ºC and 2-benzyloxirane (200 mg, 1.49 mmol, 1.0 equiv.) was added dropwise, solved in a little amount of dry THF. The reaction was stirred at -78 ºC until analysis via TLC showed complete formation of alcohol **G.** It was quenched by addition of a mixture of n-hexane:ethyl ether:saturated aqueous $NH_4Cl$ (2:2:1), being the volume of this mixture = 25 times the millimole of allyltriphenylsilane and extracted with ethyl ether. The combined organic layers were dried over anhydrous $MgSO_4$, filtered and the solvent was removed under reduced pressure. This crude reaction mixture was purified by flash silica gel column chromatography (n-hexane/EtOAc 90:10 solvent system) to afford 275 mg of alcohol **G** (1.11 mmol, 74% yield).

### 2,5-*trans*-5-benzyl-2-methyltetrahydrofuran-3-yl)trimethylsilane (SPL-5)

To a well-stirred solution of *bis*-homoallylsilyl alcohol **G** (100 mg, 0.40 mmol, 1.0 equiv.) in 4.0 mL of dry DCM (0.1 M) at 10 ºC under $N_2$ atmosphere, was added $InCl_3$ (8.8 mg, 0.040 mmol, 0.10 equiv.). Once the reaction was complete, the process was quenched by addition of water. The aqueous phase was extracted with DCM. The combined organic layers were dried over anhydrous $MgSO_4$, filtered and the solvent was removed under reduced pressure. The crude reaction mixture was purified by flash silica gel column chromatography (n-hexane/EtOAc 95:5 solvent system) to afford 47 mg of **SPL-5** (0.19 mmol, 47 % yield).

**$^1$H-NMR ($CDCl_3$, 400 MHz)** (diastereoisomeric mixture (1:0.7)) δ 7.28 (m, 3H), 7.21 (m, 5H), 4.13 (m, 1H), 4.05 (m, 1H), 3.97 (dq, *J* = 10.0 & 6.0 Hz, 1H), 3.79 (dq, *J* = 10.3 & 5.9 Hz, 1H), 3.05-2.99 (dd, *J* = 13.4 & 5.5 Hz, 1H), 2.99-2.92 (dd, *J* = 13.5 & 5.7 Hz, 1H), 2.67 (ddd, *J* = 13.3, 7.5 & 3.5 Hz, 2H), 1.97 (ddd, *J* = 12.2, 6.8 & 5.3 Hz, 1H), 1.79 (m, 2H), 1.43 (dt, *J* = 12.6 & 9.3 Hz, 1H), 1.29 (d, *J* = 6.1 Hz, 3H), 1.25 (d, *J* = 6.1 Hz, 2H), 0.97 (m, 2H), 0.00 (s, 15H).)

**$^{13}$C-NMR ($CDCl_3$, 100 MHz)** (diastereoisomeric mixture (1:0.7)) δ 139.0 (C), 138.9 (C), 129.3 (3 x CH), 129.2 (2 x CH), 128.2 (3 x CH), 126.1 (2 x CH), 79.4 (CH), 79.2 (CH), 78.2 (CH), 77.3 (CH), 42.8 ($CH_2$), 42.1 ($CH_2$), 36.1 ($CH_2$), 35.7 (CH), 34.2 ($CH_2$), 33.2 (CH), 22.3 ($CH_3$), 21.9 ($CH_3$), -2.6 (6 x $CH_3$).

**HRMS (ESI$^+$):** m/z $[M+Na]^+$ cald. for $C_{15}H_{24}ONaSi$: 271.1494; found: 271.1491.

## Example 5: Synthesis of compound SPL-6

This compound (SPL-6) was synthesized in one chemical steps from the unsaturated tris-homoallylsilyl alcohol

**H** with a yield of 47% (Scheme 5).

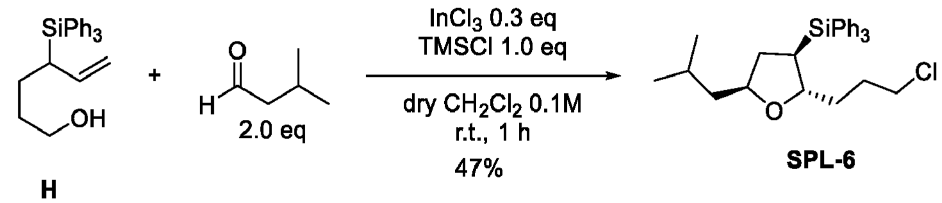


**H** **SPL-6**

**Scheme 5.** Synthesis of tri-substituted five-membered ring oxacycle **SPL-6** (2,3,5-*cis*-2-(3-choropropyl)-5-isobutyltetrahydrofuran-3-yl)triphenylsilane).

**2,3,5-*cis*-2-(3-choropropyl)-5-isobutyltetrahydrofuran-3-yl)triphenylsilane (SPL-6)**

To a well-stirred and open-air solution of *tris*-homoallylsilyl alcohol **H** (100 mg, 0.28 mmol, 1.0 equiv) in dry dichloromethane (DCM) (0.1 M) at room temperature were added isovaleraldehyde (2.0 equiv) and the $FeBr_3$ (8.7 mg, 0.030 mmol, 0.3 equiv.). The reaction was monitored by TLC, and once complete, the process was quenched by the addition of the same amount of water as DCM. The aqueous phases were separated and washed with DCM. The combined organic layers were dried over anhydrous magnesium sulfate and filtered, and the solvent was removed under reduced pressure. The crude reaction was purified by flash silica gel column chromatography (n-hexane/EtOAc 95:5 solvent system) to afford 52 mg of **SPL-6** (0.112 mmol, 47 % yield).

**$^1$H-NMR ($CDCl_3$, 400 MHz)** $\delta$ 7.60-7.50 (m, 6H), 7.5-7.3 (m, 9H), 4.2 (td, *J* = 8.6 & 2.8 Hz, 1H), 4.00-3.95 (m, 1H), 3.50-3.33 (m, 2H), 2.3 (ddd, *J* = 12.0, 8.1 & 5.1 Hz, 1H), 1.9 (dt, *J* = 11.8 & 8.1 Hz, 1H), 1.90-1.80 (m, 1H), 1.80-1.60 (m, 3H), 1.50-1.30 (m, 3H), 1.2 (ddd, *J* = 13.2, 7.3 & 5.6 Hz, 1H), 0.9 (dd, *J* = 6.6 & 3.2 Hz, 6H).

**$^{13}$C-NMR ($CDCl_3$, 100 MHz)** $\delta$ 136.1 (6 $\times$ CH), 134.0 (3 $\times$ C), 129.8 (3 $\times$ CH), 128.1 (6 x CH), 80.3 (CH), 76.8 (CH), 45.2 (CH2), 44.4 (CH2), 37.0 (CH2), 34.4 (CH2), 31.3 (CH), 30.1 (CH2), 26.0 (CH), 23.1 (CH3), 22.9 (CH3).

**HRMS (ESI$^+$):** m/z [M+Na]+ calcd for C29H35ONaSiCl, 485.2043; found, 485.2047.

**Example 6: Antiviral activity of SPL-0 against transmissible gastroenteritis virus (TGEV), as determined by quantitation of virus-induced cell death**

TGEV is an alphacoronavirus that naturally infects pigs (*Sus sp.*) and is commonly used as a model coronavirus. The effect of **SPL-0** on cellular viability in the presence or absence of TGEV infection was determined in swine testis (ST) cells inoculated with TGEV at a multiplicity of infection (MOI) of 0.01 infectious particles per cell. Real-time automated microscopy was used for determining cellular permeability to a commercial cyanine that fluorescently labels nucleic acids (Cytotox®). A range of **SPL-0** concentrations was tested in order to obtain a dose-response curve. The percentage of dead cells at 48 h after viral inoculation decreased as the **SPL-0** dose increased (**Figure 1a**), indicating that **SPL-0** partially reverts TGEV-induced cell death. A dose-response model of the form $D(c) = \frac{D_{max}-D_{min}}{1+\left(\frac{c}{EC_{50}}\right)^n} + D_{min}$ was fit to the experimental data by the least-squares method, where *c* is the compound concentration ($\mu$M), D is the percentage of dead cells, $D_{max}$ is the top plateau, $D_{min}$ is the bottom plateau, $EC_{50}$ is the half-maximal effective concentration, and *n* is Hill slope. The estimated $EC_{50}$ of **SPL-0** on TGEV-induced cell death was 13.1 $\mu$M (coefficient of determination: $r^2$ = 0.999; Hill slope: n = 2.87), with a bottom plateau of 16.1% dead cells. In cell cultures not inoculated with TGEV, the percentage of dead cells at 48 h remained <12% (range 2.5-12.0%), and showed no correlation with **SPL-0** concentration (**Figure 1b**). Therefore, **SPL-0** exhibited no detectable cytotoxic effects in the examined dose range (2-100 $\mu$M), showing that the half-maximal cytotoxic concentration ($CC_{50}$) was higher than 100 $\mu$M.

**Example 7: Antiviral activity of SPL-4 against TGEV, as determined by quantitation of virus-induced cell death**

The effect of **SPL-4** on cellular viability in the presence or absence of TGEV infection was determined in ST cells inoculated with TGEV at an MOI of 0.01 infectious particles per cell. Real-time automated microscopy was used for determining cellular permeability to Cytotox®. A range of **SPL-4** concentrations was tested in order to obtain a dose-response curve. The percentage of dead cells at 48 h after viral inoculation decreased as the **SPL-4** dose increased (**Figure 2a**), indicating that **SPL-4** partially reverts TGEV-induced cell death. The estimated $EC_{50}$ of **SPL-4** on TGEV-induced cell death was 15.8 μM ($r^2$ = 0.999, n = 2.52), with a bottom plateau of 11.4% dead cells. Therefore, the activity of **SPL-4** against TGEV was similar to that of **SPL-**0. In cell cultures not inoculated with TGEV, the percentage of dead cells at 48 h remained <12 % (range 3.1-12.0 %), and showed no correlation with **SPL-4** dose **(Figure 2b),** indicating that **SPL-4** had no detectable cytotoxic effects in the examined dose range (2-100 μM).

**Example 8: Antiviral activity of SPL-6 against TGEV, as determined by quantitation of virus-induced cell death**

The effect of **SPL-6** on cellular viability in the presence or absence of TGEV infection was determined in ST cells inoculated with TGEV at an MOI of 0.01 infectious particles per cell. Real-time automated microscopy was used for determining cellular permeability to Cytotox®. A range of **SPL-6** concentrations was tested in order to obtain a dose-response curve. The percentage of dead cells at 48 h after viral inoculation decreased as the **SPL-6** dose increased (**Figure 3a**), indicating that **SPL-6** partially reverts TGEV-induced cell death. The estimated $EC_{50}$ of **SPL-6** on TGEV-induced cell death was 30.5 μM ($r^2$ = 0.999, n = 2.13), with a bottom plateau of 14.8% dead cells. Therefore, **SPL-6** showed a less potent activity against TGEV than **SPL-0** or **SPL-4.** In cell cultures not inoculated with TGEV, the percentage of dead cells at 48 h remained <8 % (range 6.1-7.6 %), and showed no correlation with **SPL-6** dose **(Figure 3b),** indicating that **SPL-6** had no detectable cytotoxic effects in the examined dose range (2-100 μM).

**Example 9: Lack of antiviral activity of SPL-1 against TGEV, as determined by quantitation of virus-induced cell death**

The effect of **SPL-1** on cellular viability in the presence or absence of TGEV infection was determined in ST cells inoculated with TGEV at an MOI of 0.01 infectious particles per cell. Real-time automated microscopy was used for determining cellular permeability to Cytotox®. A range of **SPL-1** concentrations was tested in order to obtain a dose-response curve. **SPL-1** did not revert TGEV-induced cell death (**Figure 4a).** In cell cultures not inoculated with TGEV, the percentage of dead cells at 48 h remained <10 % (range 3.3-9.6 %). However, the percentage of dead cells increased slightly at the highest assayed dose (100 μM: **Figure 4b**).

**Example 10: Lack of antiviral activity of SPL-5 against TGEV, as determined by quantitation of virus-induced cell death**

The effect of **SPL-5** on cellular viability in the presence or absence of TGEV infection was determined in ST cells inoculated with TGEV at an MOI of 0.01 infectious particles per cell. Real-time automated microscopy was used for determining cellular permeability to Cytotox®. A range of **SPL-5** concentrations was tested in order to obtain a dose-response curve. **SPL-5** did not revert TGEV-induced cell death (**Figure 5a). SPL-0** and **SPL-5** differ in that the tri-phenylsilyl group of **SPL-0** has been replaced by a tri-methylsilyl group in **SPL-5.** Therefore, the tri-phenylsilyl group shown in formula (I) is necessary for antiviral activity. In cell cultures not inoculated with TGEV, the percentage of dead cells at 48 h remained <12 % (range 3.5-11.9 %), and showed no correlation with **SPL-5** dose (**Figure 5b**), indicating that **SPL-5** had no detectable cytotoxic effects in the examined dose range (2-100 μM).

**Example 11: Antiviral activity of SPL-0 against TGEV as determined by viral titration**

ST cells were inoculated with TGEV at a MOI of 0.01 infectious particles per cell and, after 24 h of incubation under standard culture conditions, the culture medium was subjected to the plaque assay, which determines the number of viral infectious particles present per volume unit (viral titer). A range of **SPL-0** concentrations was tested in order to obtain a dose-response curve. The TGEV titer in the harvested medium decreased as the **SPL-0** dose increased (**Figure 6**). The estimated $EC_{50}$ of **SPL-0** against TGEV was 15.6 μM ($r^2$ = 0.949, n = 1.60), with a bottom plateau of 0% for the estimated viral titer.

**Example 12: Antiviral activity of SPL-0 against TGEV quantified by reverse transcription followed by quantitative polymerase chain reaction (RT-qPCR)**

ST cells were inoculated with TGEV at a MOI of 0.01 infectious particles per cell and, after 24 h of incubation under standard culture conditions, total RNA was extracted and TGEV RNA was quantified by RT-qPCR. A range of **SPL-0** concentrations was tested in order to obtain a dose-response curve. The amount of TGEV RNA decreased as the **SPL-0** dose increased (**Figure 7**). The $EC_{50}$ of **SPL-0** against TGEV was 4.35 μM ($r^2$ = 0.951, n = 1.16), with a bottom plateau of 0 % viral RNA.

**Example 13: Antiviral activity of SPL-0 against murine hepatitis virus (MHV) quantified by RT-qPCR**

MHV is a betacoronavirus that naturally infects mice (*Mus musculus*) and is commonly used as a model coronavirus. It belongs to the same taxonomic genus as SARS-CoV-2 (betacoronaviruses). Mouse liver CCL9.1 cells were inoculated with MHV at a MOI of 0.01 infectious particles per cell and, after 24 h of incubation under standard culture conditions, total RNA was extracted and MHV RNA was quantified by RT-qPCR. A single dose of **SPL-0** (20 μM) was tested to check for antiviral effects that reproduce the results obtained with the TGEV model. The amount of MHV RNA in cells treated with 20 μM **SPL-0** was 17.0 ± 2.4 % of the amount obtained in control cultures receiving no drug, indicating that **SPL-0** is active against MHV with an $EC_{50}$ < 20 μM.

**Example 14: Antiviral activity of SPL-0 against human coronavirus OC43 quantified by RT-qPCR**

OC43 betacoronavirus is a causative agent of human common cold. It belongs to the same taxonomic genus as SARS-CoV-2 (betacoronaviruses). OC43 conveniently infects BHK-21 baby hamster kidney cells under laboratory conditions. BHK-21 cells were inoculated with OC43 coronavirus at a MOI of 0.01 infectious particles per cell and, after 4 days of incubation under standard culture conditions, total RNA was extracted and OC43 RNA was quantified by RT-qPCR. A single dose of **SPL-0** (20 μM) was tested to check for antiviral effects that reproduce the results obtained with other coronaviruses. The amount of OC43 RNA in cells treated with 20 μM **SPL-0** was 4.16 ± 0.95 % of the amount obtained in control cultures receiving no drug, indicating that **SPL-0** is active against OC43 with an $EC_{50}$ < 20 μM.

**Example 15: Antiviral activity of SPL-0 against SARS-CoV-2 quantified by immunofluorescence in African green monkey cells**

To determine the activity of **SPL-0** against SARS-CoV-2, a dose-response assay was performed in the range of 0.2 μM to 50 μM using VeroE6 kidney cells from African green monkey (*Cercopithecus aethiops*). Cell cultures were inoculated with SARS-CoV-2 at an MOI of 0.01 infectious particles per cell and analyzed after 48 h. The percentage of infected cells was obtained by flow cytometry using a monoclonal antibody against the virus spike protein S as well as a fluorescently labelled secondary antibody (immunofluorescence). The percentage of infected cells decreased as the **SPL-0** increased (**Figure 8a**). The $EC_{50}$ of **SPL-0** against SARS-CoV-2 in VeroE6 cells was 27.5 μM ($r^2$ = 0.993, n = 4.58), with a bottom plateau of 1.6 % infected cells. In addition, for each dose, cell viability assays were performed in the absence of virus. For this, the MTT assay, a standard procedure for determining cell metabolic activity, was used. **SPL-0** showed no cytotoxic effects over the range of concentrations tested **(Figure 8b).** The percentage of viable cells at 48 h remained >85 % (range 85.0-93.3%), and showed no correlation with **SPL-0** dose.

**Example 16: Antiviral activity of SPL-0 against SARS-CoV-2 quantified by immunofluorescence in human lung cells**

The activity of **SPL-0** against SARS-CoV-2 was determined in A549 human lung cells, which provide a biologically relevant model, since that lungs are a primary target of SARS-CoV-2. For this, a dose-response assay was performed in the range of 0.2 μM to 50 μM. Cell cultures were inoculated with SARS-CoV-2 at an MOI of 0.01 infectious particles per cell and analyzed after 72 h. The percentage of infected cells was obtained by flow cytometry using a monoclonal antibody against the virus spike protein S as well as a fluorescently labelled secondary antibody (immunofluorescence). The percentage of infected cells decreased as the **SPL-0** increased (**Figure 9a**). The $EC_{50}$ of **SPL-0** against SARS-CoV-2 in A549 cells was 12.9 μM ($r^2$ = 0.994, n = 3.48), with an estimated bottom plateau of -0.37 % (i.e. effectively no infection). In addition, for each dose, cell viability assays were performed in the absence of virus. For this, the MTT assay was used. **SPL-0** showed no cytotoxic effects over the range of concentrations tested (**Figure 9b**). The percentage of viable cells at 48 h remained >89 % (range 89.0-100 %), and showed no correlation with **SPL-0** dose.

**Example 17: Antiviral activity of SPL-0 against coxsackievirus B3 (CVB3), as determined by viral titration**

CVB3 belongs to the picornavirus family. Hence, CVB3 is not a coronavirus. Coronaviruses and picornaviruses share certain broad features. Both are plus-strand RNA viruses. However, they are otherwise largely unrelated virus groups. The effect of **SPL-0** on CVB3 infection was determined in human HeLa cells. HeLa cells were inoculated with CVB3 at a MOI of 0.01 infectious particles per cell and, after 24 h of incubation under standard culture conditions, the medium was subjected to the plaque assay, which determines the number of viral infectious particles present per volume unit (viral titer). A range of **SPL-0** concentrations was to obtain a dose-response curve. The CVB3 titer in the harvested medium decreased as the **SPL-0** dose increased (**Figure 10**). The $EC_{50}$ of **SPL-0** against CVB3 was 2.95 μM ($r^2$ = 0.980, n = 0.828), with a bottom plateau of 0 % for the estimated viral titer. Therefore, the antiviral action of **SPL-0**

extends beyond coronaviruses.

**Table 1** and **Table 2** show a summary of the results:

**Table 1.** Summary of the activity of different compounds against TGEV as determined by quantitation of virus-induced cell death

| | | |
|---|---|---|
| SPL-0 | 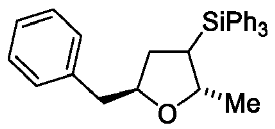 | **Active ($EC_{50}$ = 13.1 μM)** |
| SPL-1 | 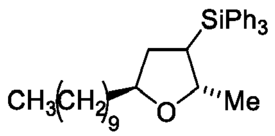 | Inactive |
| SPL-4 | 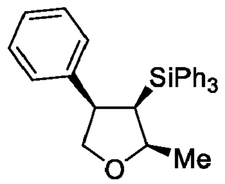 | Active ($EC_{50}$ = 15.8 μM) |
| SPL-5 | 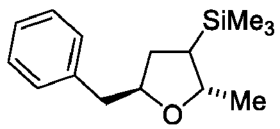 | Inactive |
| SPL-6 | 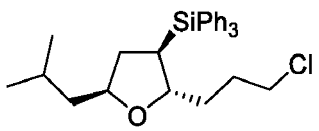 | Active ($EC_{50}$ = 30.5 μM) |

**Table 2.** Summary of the activity of SPL-0 against different viruses by different methods

| **Virus** | **Cells** | **Method** | **$EC_{50}$** | **$CC_{50}$** |
|---|---|---|---|---|
| TGEV | ST | Virus-induced cell death | 13.1 μM | >100 μM |
| TGEV | ST | Viral titration | 15.6 μM | ND |
| TGEV | ST | RT-qPCR | 4.35 μM | ND |
| MHV | CCL9.1 | RT-qPCR | <20 μM | ND |
| OC43 | BHK-21 | RT-qPCR | <20 μM | ND |
| SARS-CoV-2 | VeroE6 | Inmunofluorescence | 27.5 μM | >50 μM |
| SARS-CoV-2 | A549 | Inmunofluorescence | 12.9 μM | >50 μM |
| CVB3 | HeLa | Viral titration | 2.95 μM | ND |
| ND: not determined | | | | |

## Claims

1. A compound of formula (I):

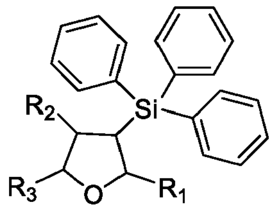

(I)

an isomer or a solvate thereof, wherein

$R_1$ is selected from $CH_3$ and $(CH_2)_nX$, being n and integer between 1 and 4 and X an halogen,
$R_2$ is selected from H and phenyl,
$R_3$ is selected from H, phenyl, benzyl and $C_1$-$C_4$ alkyl,
wherein one of $R_2$ and $R_3$ is H and the other one is different from H and with the proviso that when $R_3$ is $C_1$-$C_4$ alkyl, $R_1$ is $(CH_2)_nX$.

**2.** The compound according to claim 1, wherein $R_1$ is $CH_3$.

**3.** The compound, according to any of the previous claims, wherein $R_2$ is H and $R_3$ is selected from phenyl and benzyl.

**4.** The compound, according to claim 1, wherein $R_3$ is $C_1$-$C_4$ alkyl and $R_1$ is $(CH_2)_nX$, being n equals 3 and/or X is Cl.

**5.** The compound, according to claim 1 or 2, wherein $R_3$ is H and $R_2$ is phenyl.

**6.** The compound, according to any of claims 1 to 4, wherein the compound of formula (I) is a diastereomer selected form:

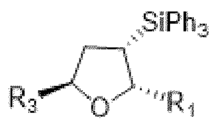 , 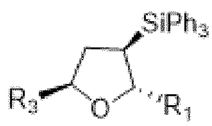

(I')    (I'')

or combinations thereof, wherein Ph represents a phenyl group.

**7.** The compound, according to claim 1, 2 or 5, wherein the compound is the isomer of formula (I'''):

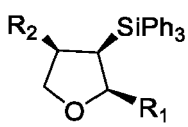

(I''')

wherein Ph represents a phenyl group.

**8.** The compound, according to claim 1, wherein the compound is selected from the next ones:

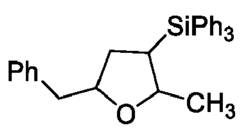 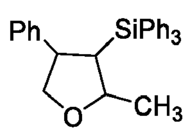 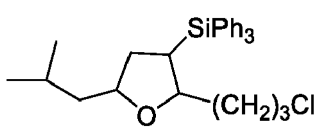

wherein Ph represents a phenyl group.

**9.** The compound according to claim 1 or 8, that is selected from the next isomers:

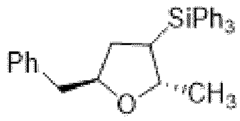
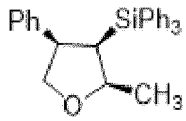
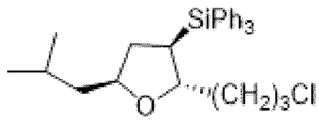

wherein Ph represents a phenyl group.

**10.** The compound of formula (I), as defined in any of claims 1-9, for use as a medicament.

**11.** A compound of formula (I), as defined in any of claims 1-9, for use as antiviral agent.

**12.** The compound of formula (I) as defined in any of claims 1-9, in the treatment and/or prevention of coronavirus infection.

**13.** The compound for use, according to claim 12, wherein the coronavirus infection is selected from: SARS-CoV-2, OC43-CoV, gastroenteritis virus (TGEV) and murine hepatitis virus (MHV) infection.

**14.** A pharmaceutical composition comprising the compound of formula (I), as defined in any of claims 1-9, and at least one excipient, adjuvant and/or a pharmaceutically acceptable carrier.

**15.** A pharmaceutical composition, according to claim 14, for use in the treatment and/or prevention of coronavirus infection.

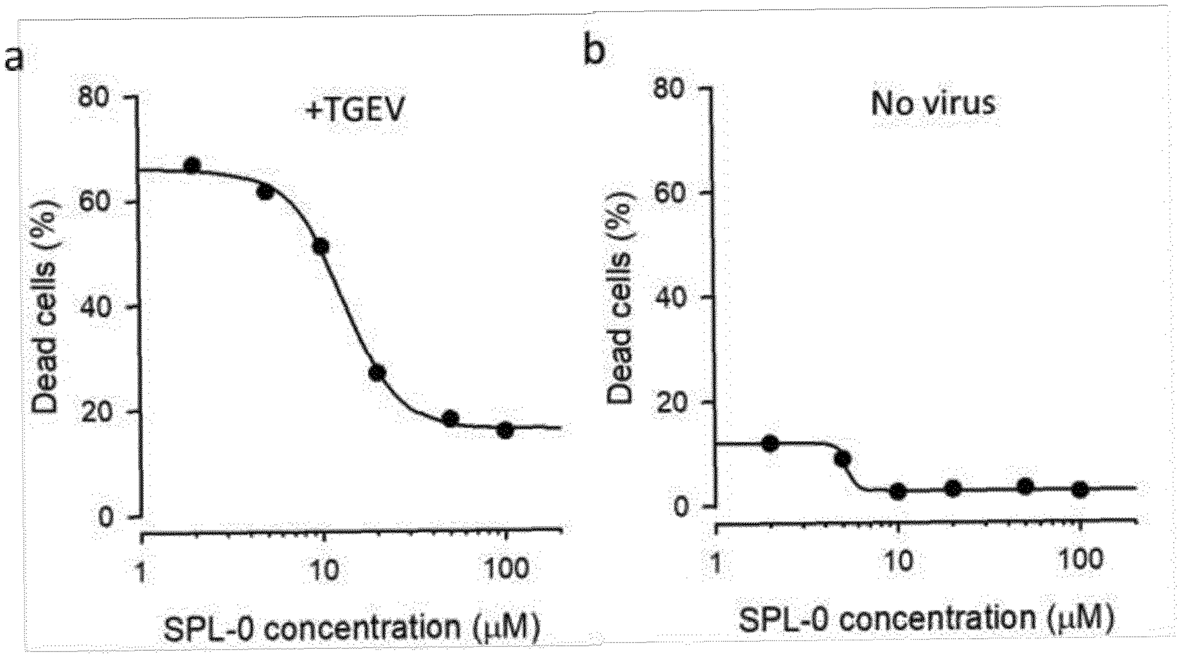
a
+TGEV
Dead cells (%)
80
60
40
20
0
1
10
100
SPL-0 concentration (μM)
b
No virus
Dead cells (%)
80
60
40
20
0
1
10
100
SPL-0 concentration (μM)

Fig. 1

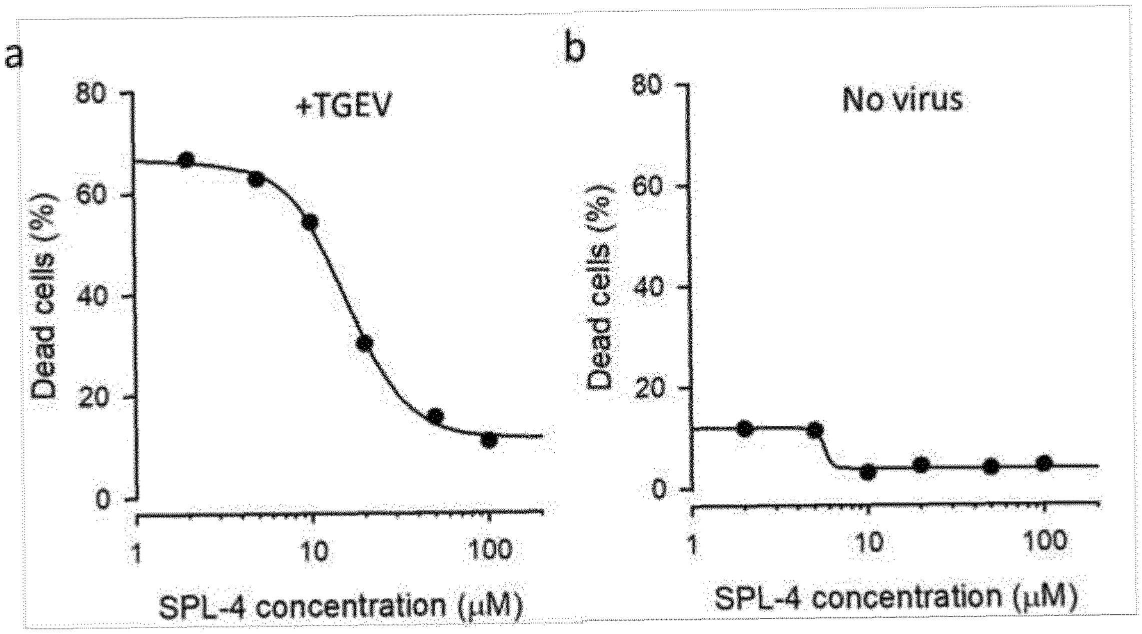
a
+TGEV
Dead cells (%)
80
60
40
20
0
1
10
100
SPL-4 concentration (μM)
b
No virus
Dead cells (%)
80
60
40
20
0
1
10
100
SPL-4 concentration (μM)

Fig. 2

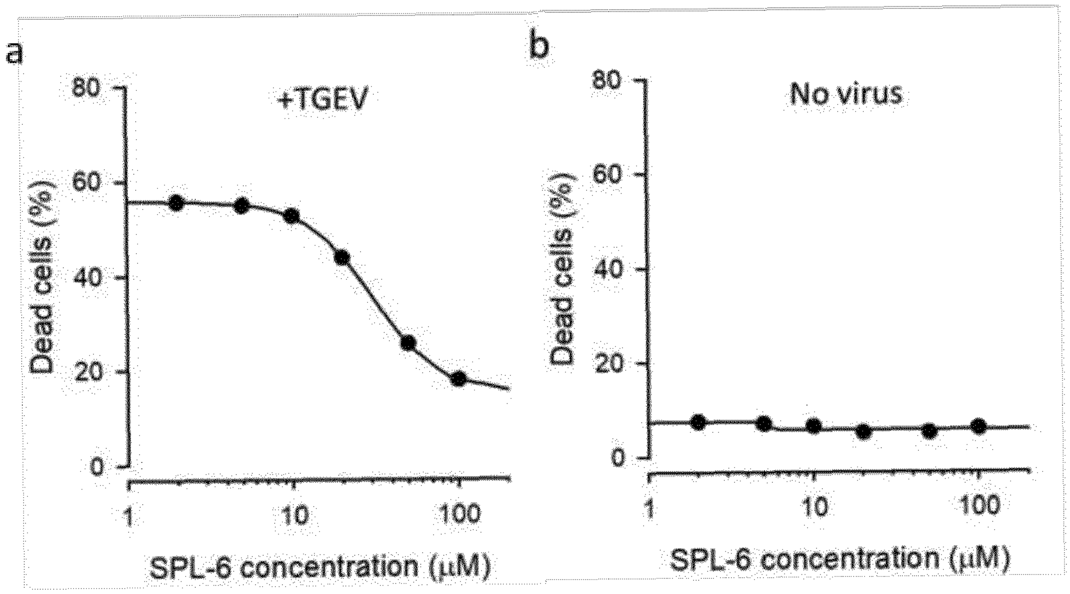
a
+TGEV
Dead cells (%)
80
60
40
20
0
1
10
100
SPL-6 concentration (μM)
b
No virus
Dead cells (%)
80
60
40
20
0
1
10
100
SPL-6 concentration (μM)

Fig. 3

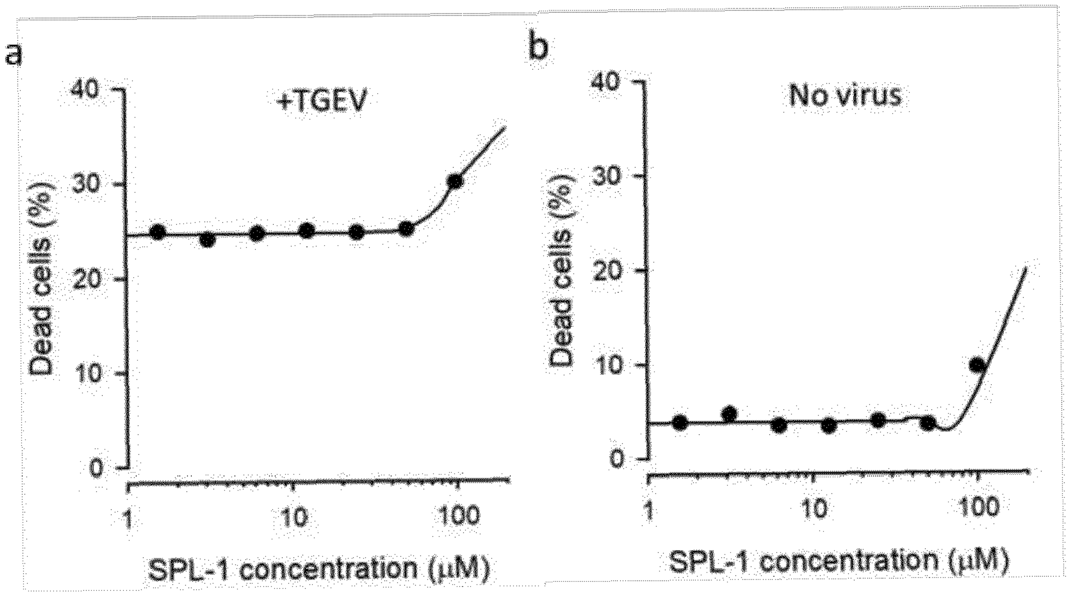
a
+TGEV
Dead cells (%)
40
30
20
10
0
1
10
100
SPL-1 concentration (μM)
b
No virus
Dead cells (%)
40
30
20
10
0
1
10
100
SPL-1 concentration (μM)

Fig. 4

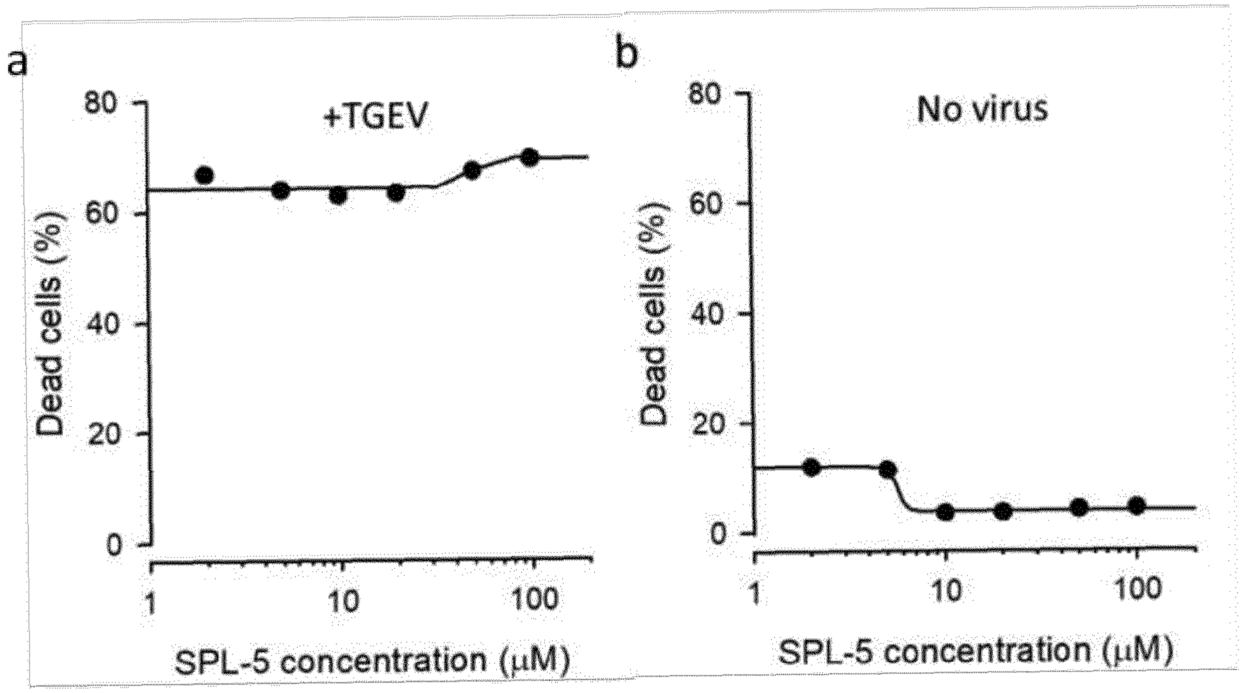
a
+TGEV
Dead cells (%)
80
60
40
20
0
1
10
100
SPL-5 concentration (μM)
b
No virus
Dead cells (%)
80
60
40
20
0
1
10
100
SPL-5 concentration (μM)

Fig. 5

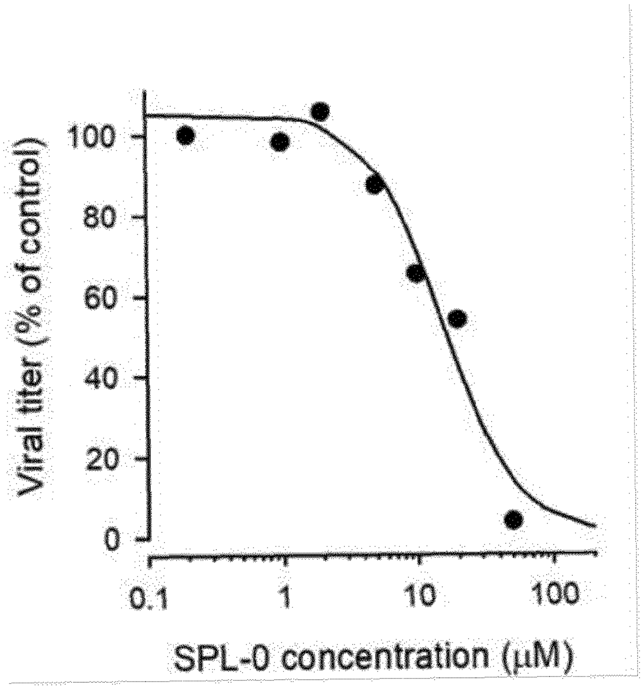
Viral titer (% of control)
100
80
60
40
20
0
0.1
1
10
100
SPL-0 concentration (μM)

Fig. 6

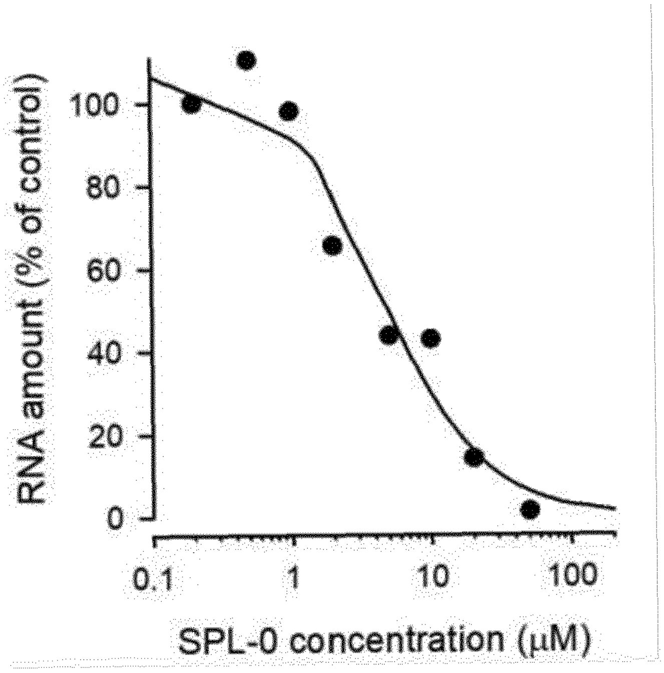
RNA amount (% of control)
100
80
60
40
20
0
0.1
1
10
100
SPL-0 concentration (μM)

Fig. 7

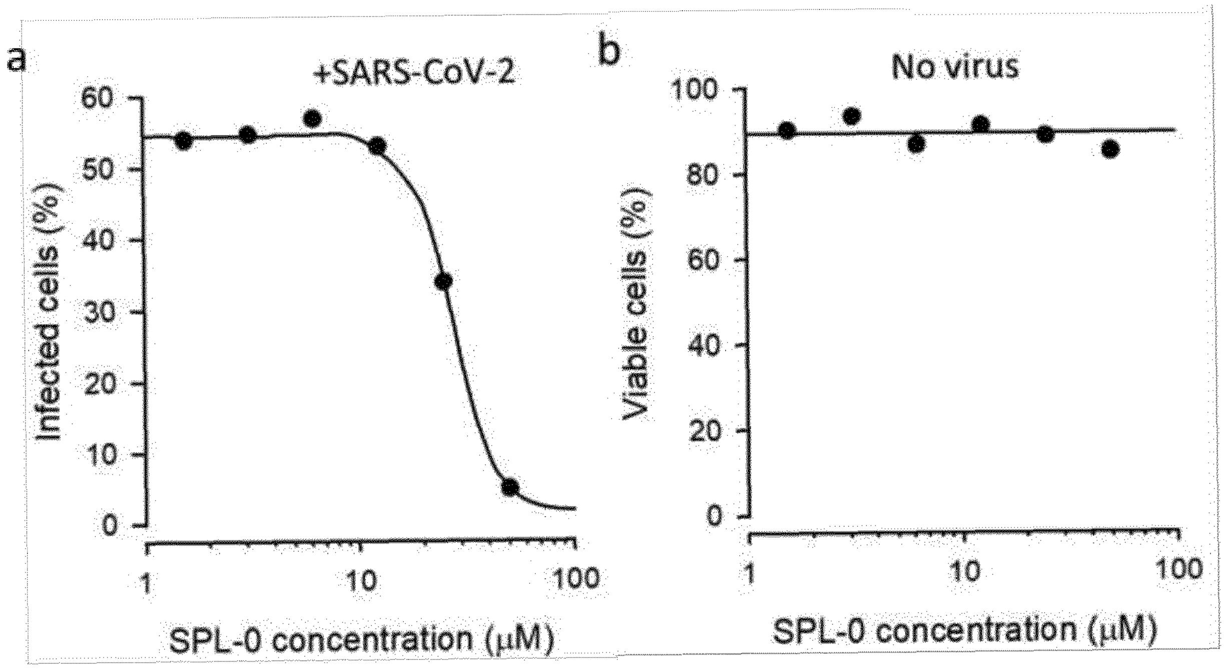
a
+SARS-CoV-2
Infected cells (%)
60
50
40
30
20
10
0
1
10
100
SPL-0 concentration (μM)
b
No virus
Viable cells (%)
100
80
60
40
20
0
1
10
100
SPL-0 concentration (μM)

Fig. 8

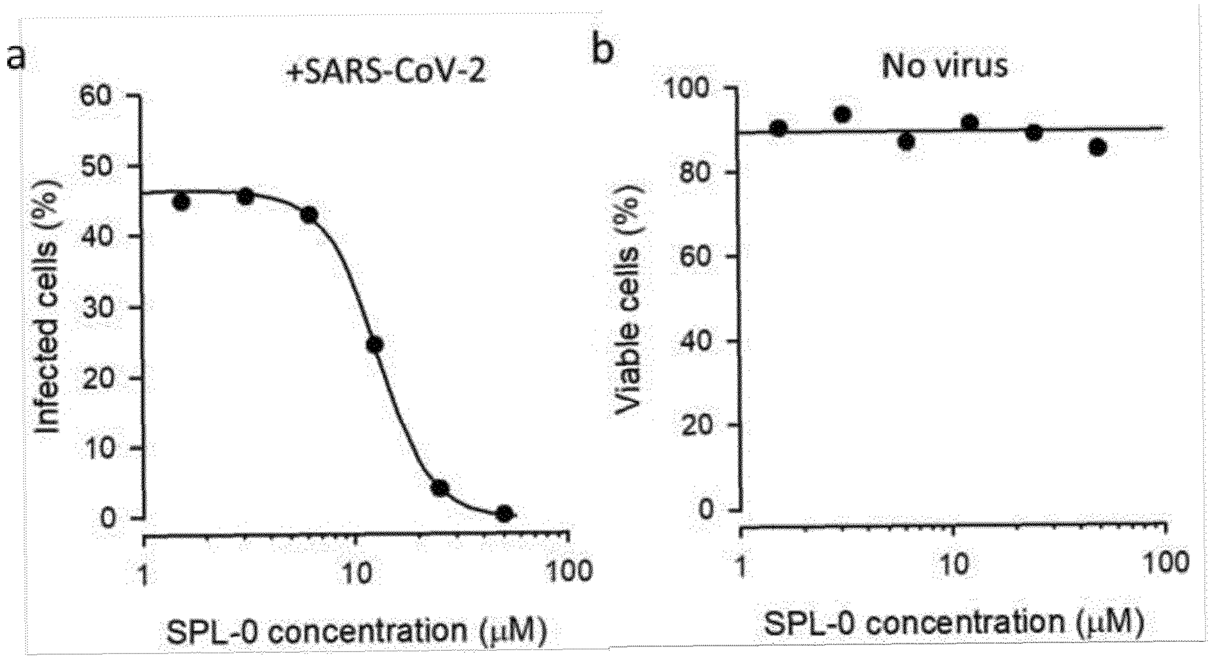
a
+SARS-CoV-2
Infected cells (%)
60
50
40
30
20
10
0
1
10
100
SPL-0 concentration (μM)
b
No virus
Viable cells (%)
100
80
60
40
20
0
1
10
100
SPL-0 concentration (μM)

Fig. 9

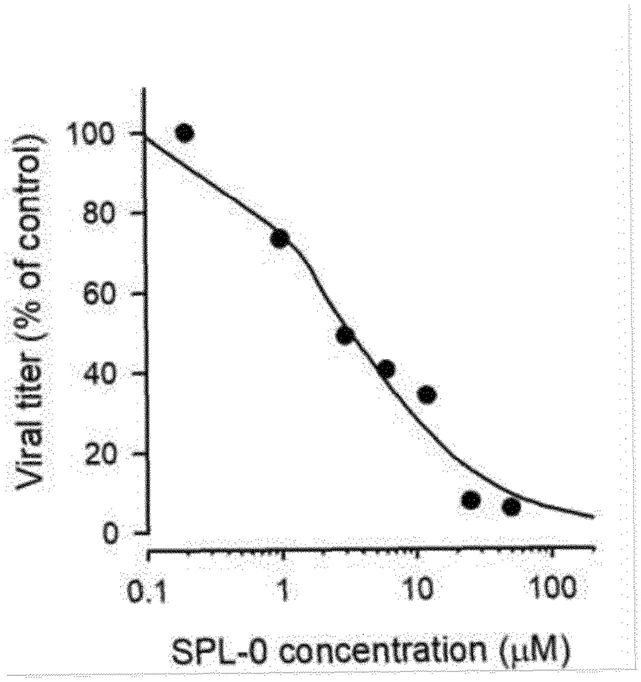
Viral titer (% of control)
100
80
60
40
20
0
0.1
1
10
100
SPL-0 concentration (μM)

Fig. 10

Europäisches Patentamt
European Patent Office
Office européen des brevets

# EUROPEAN SEARCH REPORT

Application Number
EP 21 38 2920

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | ADIWIDJAJA GUNADI ET AL: "Electrophile-induced cyclizations of silyl-substituted 4-alken-1-ols", LIEBIGS ANNALEN: ORGANIC AND BIOORGANIC CHEMISTRY., vol. 1995, no. 3, 1 March 1995 (1995-03-01), pages 501-507, XP055893352, US ISSN: 0947-3440, DOI: 10.1002/jlac.199519950369 Retrieved from the Internet: URL:http://dx.doi.org/10.1002/jlac.199519950369> * compounds rac-27 * ----- | 1,6,14 | INV. C07F7/08 A61K31/695 A61P31/12 |
| X | Corriu R: "SYNTHESES A PARTIR DE CARBANlONS ALLYLIQUES SILIClES I. CARBANIONS DERIVES DE MONOALLYLSILANES", Journal of Organomeiallic Chemistry, 1 January 1975 (1975-01-01), pages 71-80, XP055893353, Retrieved from the Internet: URL:https://www.sciencedirect.com/science/article/pii/S0022328X00941457/pdf?md5=47dff459c2afb9e8f66a9c4189f3e43d&pid=1-s2.0-S0022328X00941457-main.pdf [retrieved on 2022-02-18] * compound (X) * ----- | 1,14 | TECHNICAL FIELDS SEARCHED (IPC) A61P C07F A61K |
| A | WO 98/11073 A1 (UPJOHN CO [US]; VAILLANCOURT VALERIE A [US] ET AL.) 19 March 1998 (1998-03-19) * examples 41-45 * * claim 13 * * abstract * * table 4 * ----- | 1-15 | |

1 The present search report has been drawn up for all claims

| Place of search | Date of completion of the search | Examiner |
|---|---|---|
| The Hague | 18 February 2022 | Eberhard, Michael |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO. EP 21 38 2920

This annex lists the patent family members relating to the patent documents cited in the above-mentioned European search report. The members are as contained in the European Patent Office EDP file on
The European Patent Office is in no way liable for these particulars which are merely given for the purpose of information.

18-02-2022

| Patent document cited in search report | Publication date | Patent family member(s) | Publication date |
|---|---|---|---|
| WO 9811073 A1 | 19-03-1998 | AU 4172197 A | 02-04-1998 |
| | | CA 2262786 A1 | 19-03-1998 |
| | | EP 0927164 A1 | 07-07-1999 |
| | | JP 2002505660 A | 19-02-2002 |
| | | US 6211376 B1 | 03-04-2001 |
| | | US 6252080 B1 | 26-06-2001 |
| | | US 6310211 B1 | 30-10-2001 |
| | | US 6500842 B1 | 31-12-2002 |
| | | WO 9811073 A1 | 19-03-1998 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **PIZZORNO, A. et al.** In vitro evaluation of antiviral activity of single and combined repurposable drugs against SARS-CoV-2. *Antiviral Res,* 2020, vol. 181, 104878
- **WHITE, K. M. et al.** Plitidepsin has potent preclinical efficacy against SARS-CoV-2 by targeting the host protein eEF1A. *Science,* 2021, vol. 371, 926-931
- **CRUZ, D. A. ; SINKA, V. ; MARTIN, V. S. ; PADRÓN, J. I.** *J. Org. Chem.,* 2018, vol. 83, 12632-12647